# EUROPEAN PATENT APPLICATION

(11) **EP 4 070 821 A1**
(43) Date of publication of application: **12.10.2022**
(21) Application number: 21821480.7
(22) Date of filing: 01.04.2021
(51) Int. Cl.: A61K 48/00, A61K 31/7105, A61K 45/06, A61P 21/00, A23L 33/13, A23K 20/153

(54) **COMPOSITION FOR PREVENTING OR TREATING DISEASE CAUSED BY MUSCLE LOSS, COMPRISING AGENT INHIBITING PHF20**

(30) Priority: 11.06.2020 KR 20200070987
(71) Applicant: Mitos Therapeutics Inc., Daejeon 35015 (KR)
(72) Inventor: PARK, Jong Sun, Daejeon 35019 (KR); PARK, Ji Soo, Daejeon 35019 (KR); LEE, Hyun Ji, Daejeon 35398 (KR)
(74) Representative: Cabinet Becker et Associés
(86) International application number: PCT/KR2021/004056
(87) International publication number: WO 2021/251602

(57) **Abstract**

The present invention relates to a function of PHF20 (PHD finger protein 20) in a myogenic differentiation mechanism and, more particularly, to a use of a PHF20 gene expression inhibitor or a PHF20 protein activity inhibitor. The PHF20 gene expression inhibitor or the PHF20 protein activity inhibitor according to the present invention promotes muscle differentiation in vitro and in vivo, and thus can be utilized in various ways in the field of prevention, improvement or treatment of diseases caused by muscle loss.

## Description

### [Technical Field]

The present invention relates to the function of PHD finger protein 20 (PHF20) in a myogenic differentiation mechanism, and more particularly, to the use of a PHF20 gene expression inhibitor or a PHF20 protein activity inhibitor.

### [Background Art]

The mass of skeletal muscle is 40% to 50% of the human body weight and is the tissue having the largest mass in the body. The development of skeletal muscle requires mechanisms that include myogenic lineage commitment, myoblast proliferation, and terminal differentiation. The process of myogenesis serves as terminal effectors of the signaling cascade and is regulated by appropriate developmental stage-specific transcripts. Differentiation stages are controlled by a complex network of muscle-specific transcription factors, including the myogenic differentiation marker (MyoD) family, the myocyte enhancer factor-2 (MEF2) family, and other transcription factors.

Sarcopenia, which is caused by a decrease in muscle, is mainly caused by a decrease in muscle due to aging. Currently, since aging is emerging as an important problem not only in Korea but also around the world, social interest in sarcopenia is growing. To reflect this, the United States assigned a disease code (M62.84) to senile sarcopenia in the World Health Organization (WHO) disease classification system in October 2016. However, there are no approved treatments so far, and there are very few drugs under development compared to other diseases. In particular, most drugs targeting the myostatin-activin-follistatin system and androgen receptor have limitations in that the therapeutic effect is not large or there is a fatal side effect. Thus, there is a need for research on therapeutic drugs of sarcopenia.

### [Disclosure]

### [Technical Problem]

Accordingly, the present inventors completed the present invention by elucidating the mechanism of PHF20 in myogenic differentiation.

An object of the present invention is to provide a pharmaceutical composition for preventing or treating diseases caused by muscle loss, the composition including a PHF20 gene expression inhibitor or a PHF20 protein activity inhibitor as an active ingredient.

Another object of the present invention is to provide a pharmaceutical composition for preventing or treating diseases caused by muscle loss, the composition including a PHF20 gene expression inhibitor or a PHF20 protein activity inhibitor; or a YY1 promoter activity inhibitor.

Still another object of the present invention is to provide a composition for increasing muscle or inhibiting muscle loss, the composition including a PHF20 gene expression inhibitor or a PHF20 protein activity inhibitor as an active ingredient.

Yet another object of the present invention is to provide a method for treating a disease caused by muscle loss, the method including step of treating an individual in need thereof with a PHF20 gene expression inhibitor or a PHF20 protein activity inhibitor.

### [Technical Solution]

In order to achieve the above object, the present invention provides a pharmaceutical composition for preventing or treating a disease caused by muscle loss, the composition including a PHF20 (PHD finger protein 20) gene expression inhibitor or a PHF20 protein activity inhibitor as an active ingredient.

In addition, the present invention provides a pharmaceutical composition for preventing or treating a disease caused by muscle loss, the composition including a PHF20 gene expression inhibitor or a PHF20 protein activity inhibitor; or a YY1 promoter activity inhibitor.

In addition, the present invention provides a health functional food composition for increasing muscle or inhibiting muscle loss, the composition including a PHF20 gene expression inhibitor or a PHF20 protein activity inhibitor as an active ingredient.

In addition, the present invention provides a feed additive composition for increasing muscle or inhibiting muscle loss, the composition including a PHF20 gene expression inhibitor or a PHF20 protein activity inhibitor as an active ingredient.

In addition, the present invention provides a method for treating a disease caused by muscle loss, the method including step of treating an individual in need thereof with a PHF20 gene expression inhibitor or a PHF20 protein activity inhibitor.

### [Advantageous Effects]

The PHF20 gene expression inhibitor or the PHF20 protein activity inhibitor according to the present invention promotes myogenic differentiation in vitro and in vivo so that it can be used in various ways in the field of prevention, improvement or treatment of diseases caused by muscle loss.

### [Description of Drawings]

FIG. 1 is a view showing the results of confirming the expression of PHF20 during myogenic differentiation of C₂C₁₂ cells through qRT-PCR (Pre, premyocytes; D1-D5, cells according to the number of differentiated days).
FIG. 2 is a view showing the results of confirming the expression of PHF20 and MyoD during myogenic differentiation of C₂C₁₂ cells through western blotting.
FIG. 3 is a view showing the result of confirming the expression of YY1 during myogenic differentiation of C2C12 cells through qRT-PCR.
FIG. 4 is a view showing the results of confirming the expression of YY1 and MyoD during myogenic differentiation of C2C12 cells through Western blotting.
FIG. 5 is a view showing the results of confirming the effect of PHF20 expression on the differentiation of C2C12 cells (-Doxy, doxycycline untreated cells; +Doxy, doxycycline treated cells). In more detail, FIG. 5A is a view showing the results of analyzing the protein expression of PHF20, YY1 and MyoD according to whether or not doxycycline was treated in C2C12 cells, and FIG. 5B is a view showing the results of analyzing the mRNA expression of PHF20, YY1 and MyoD according to whether or not doxycycline was treated in C2C12 cells.
FIG. 6 is a view showing the results of confirming the effect of the expression of PHF20 on myotube formation through immunochemical staining analysis.
FIG. 7 is a view showing the results of confirming the effect of inhibition of PHF20 expression on the expression of YY1 and MyoD through Western blotting. In more detail, FIG. 7A is a view showing the results of C2C12 cells before differentiation (Pre), and FIG. 7B is a view showing the results of differentiated myofibers for 1 day.
FIG. 8A is a view showing the results of confirming the expression of PHF20 according to the concentration of doxycycline treatment through Western blotting (upper panel) and the results of confirming the activity of the YY1 promoter according to the expression of the PHF20 through luciferase reporter gene analysis (lower panel).
FIG. 8B is a view showing the results of confirming the expression of PHF20 according to shRNA transfection through Western blotting (upper panel) and the result of confirming the activity of the YY1 promoter according to the expression of the PHF20 through luciferase reporter gene analysis (lower panel).
FIG. 9A is a view showing the results of confirming the activity of the YY1 promoter (upper panel) and the expression of PHF2, YY1 and MyoD (lower panel) according to the differentiation period in C₂C₁₂ cells not treated with doxycycline.
FIG. 9B is a view showing the results of confirming the activity of the YY1 promoter (upper panel) and the expression of PHF2, YY1 and MyoD (lower panel) according to the differentiation period in C₂C₁₂ cells treated with doxycycline.
FIG. 10 is a view showing the results of confirming how PHF20 regulates the YY1 promoter through chromatin immunoprecipitation analysis.
FIG. 11 is a view showing the results of confirming the effect of YY1 expression on MyoD expression in C2C12 cells (Pre) and cells differentiated for 3 days through Western blotting.
FIG. 12 is a view showing the results of confirming the effect of YY1 expression on myotube formation in C2C12 cells differentiated for 1 day through immunochemical staining analysis.
FIG. 13 is a view showing the results of confirming the expression of PHF20, YY1 and MHC in PHF20 transgenic mice through Western blotting (WT, control group; PHF20-TG, PHF20 transgenic mice).
FIG. 14A is a view showing the results of measuring the body weight of PHF20 transgenic mice.
FIG. 14B is a view showing the results of observing the thigh muscle of a PHF20 transgenic mouse.
FIG. 15 is a view showing the results of observing gastrocnemius tissues of PHF20 transgenic mice through histological analysis. In more detail, FIG. 15A is a view showing the results of observing a cross-section of the gastrocnemius tissue, and FIG. 15B is a view showing the results of observing a longitudinal cross-section thereof.
FIG. 16 is a view showing the result of additionally observing a cross-section of gastrocnemius tissue of transgenic mice through histological analysis.
FIG. 17 is a view showing the results of observing gastrocnemius tissues of PHF20 transgenic mice through immunochemical staining analysis. In more detail, FIG. 17A is a view showing the results of observing a longitudinal cross-section of the gastrocnemius tissue, and FIG. 17B is a view showing the results of observing a cross-section thereof.
FIG. 18 is a view illustrating a mechanism in which the expression of PHF20 affects myogenic differentiation.

### [Best Mode]

Hereinafter, the present invention will be described in detail.

According to an aspect of the present invention, the present invention provides a pharmaceutical composition for preventing or treating a disease caused by muscle loss, the composition including a PHF20 gene expression inhibitor or PHF20 protein activity inhibitor as an active ingredient.

In addition, the present invention provides a method for treating a disease caused by muscle loss, the method including step of treating an individual in need thereof with a PHF20 gene expression inhibitor or a PHF20 protein activity inhibitor.

In the present invention, PHF20 (plant homeodomain finger protein 20, PHD finger protein 20) is a multi-domain protein and subunit of a lysine acetyltransferase complex that acetylates histone H4. PHF20 is a transcription factor, and it was first identified in glioma patients. PHF20 knockout mice die shortly after birth, and it exhibits diverse phenotypes within the skeletal and hematopoietic system. However, the detailed mechanisms of these phenotypes in PHF20 knockout mice have not been reported.

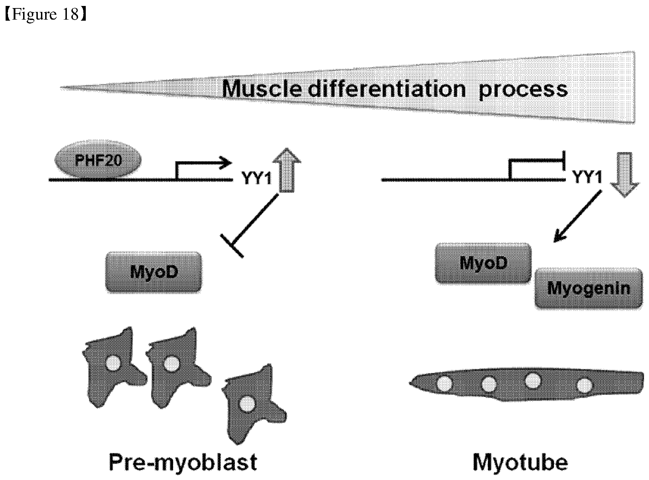

In an example of the present invention, it was confirmed that the PFH20 affects myogenic differentiation through the mechanism shown in FIG. 18. More specifically, inhibition of the PHF20 expression causes isolation of PHF20 bound to the YY1 promoter in vitro and in vivo to increase the expression of myogenic differentiation marker (MyoD), thereby promoting myogenic differentiation.

In a specific embodiment of the present invention, the PHF20 gene expression inhibitor is preferably a YY1 promoter activity inhibitor.

In a specific embodiment of the present invention, the PHF20 gene expression inhibitor may increase MyoD expression to promote myogenic differentiation.

In the present invention, YY1 (Yin Yang 1) refers to a transcriptional repression protein that binds to DNA through four C-terminal zinc finger domains. In skeletal muscle, treatment with rapamycin, an inhibitor of the mammalian target of rapamycin (mTOR), increases mitochondrial transcriptional regulator gene expression, thereby reducing mitochondrial gene expression and oxygen consumption. mTOR phosphorylation and its downstream targets YY1 and Peroxisome proliferator-activated receptor gamma coactivator 1-alpha (PGC-1α) are increased by FGF21 (fibroblast growth factor 21) treatment in C₂C₁₂ myoblasts. Activation of the mTOR-YY1-PGC1α pathway by FGF21 in myoblasts regulates intracellular ATP synthesis, oxygen consumption rate, citrate synthase activity, glycolysis, mitochondrial DNA copy number, and energy homeostasis manifested by a significant increase in the expression of major energies.

In a specific embodiment of the present invention, the disease caused by muscle loss is preferably selected from the group consisting of sarcopenia, atony, muscular atrophy, muscular dystrophy, muscle degeneration, myotonic dystrophy, amyotrophic lateral sclerosis, myasthenia and cachexia, but is not limited thereto.

In the present invention, prevention refers to any action that inhibits or delays the onset of disease due to muscle loss by administration of the pharmaceutical composition according to the present invention. In addition, in the present invention, treatment refers to any action that improves or beneficially changes the symptoms of disease due to muscle loss by administration of the pharmaceutical composition according to the present invention.

In an embodiment of the present invention, the PHF20 gene expression inhibitor may include at least one type selected from the group consisting of shRNA, siRNA, ribozyme, and antisense nucleotides complementary to the PHF20 gene.

In a preferred embodiment of the present invention, the PHF20 gene expression inhibitor may be an shRNA shown by the nucleotide sequence represented by SEQ ID NO: 1, and variants of the nucleotide sequence represented by SEQ ID NO: 1 are also included within the scope of the present invention. A functional equivalent of the shRNA represented by SEQ ID NO: 1 of the present invention is a concept including variants capable of functionally the same action as the shRNA consisting of the nucleotide sequence represented by SEQ ID NO: 1, although for example, a portion of the sequence represented by SEQ ID NO: 1 was modified by deletion, substitution, or insertion.

Specifically, the shRNA may include a sequence having at least 70%, more preferably at least 80%, still more preferably at least 90%, and most preferably at least 95% sequence homology to the base sequence represented by SEQ ID NO: 1, respectively. The "% sequence homology" for a polynucleotide or amino acid is identified by comparing the comparison region with two optimally arranged sequences.

The siRNA includes a sense sequence of 15 to 30 mers selected from the base sequence of the mRNA of the PHF20 gene and an antisense sequence complementary to the sense sequence, but the sense sequence is not particularly limited thereto.

In a specific embodiment of the present invention, the PHF20 protein activity inhibitor may include at least one type selected from the group consisting of compounds, peptides and antibodies that specifically bind to PHF20 protein.

The compound includes any compound capable of specifically binding to and inhibiting the activity of the PHF20 protein.

The antibody may specifically and directly bind to the PHF20 protein to effectively inhibit the activity of the PHF20 protein. As the antibody that specifically binds to the PHF20 protein, a polyclonal antibody or a monoclonal antibody is preferably used, and the antibody may be prepared by a known method known to those skilled in the art, and commercially known antibodies may be purchased and used.

The pharmaceutical composition of the present invention may further include a pharmaceutically acceptable additive, and the pharmaceutically acceptable additive may include starch, gelatinized starch, microcrystalline cellulose, lactose, povidone, colloidal silicon dioxide, calcium hydrogen phosphate, lactose, mannitol, syrup, Arabic gum, pregelatinized starch, corn starch, powdered cellulose, hydroxypropyl cellulose, Opadry, sodium starch glycolate, carnauba wax, synthetic aluminum silicate, stearic acid, magnesium stearate, aluminum stearate, calcium stearate, white sugar, etc. The pharmaceutically acceptable additive according to the present invention is preferably included in an amount of 0.1 to 90 parts by weight based on the composition, but is not limited thereto.

In addition, the pharmaceutical composition of the present invention may be administered in various oral or parenteral formulations during actual clinical administration. In the case of formulation, it may be prepared using commonly used fillers, extenders, binders, wetting agents, disintegrants, diluents such as surfactants, or excipients, and it is preferable to use suitable preparations known in the art. Carriers, excipients and diluents that may be included in the composition include lactose, dextrose, sucrose, oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxy benzoate, propyl hydroxy benzoate, talc, magnesium stearate, mineral oil, and the like.

The solid preparation for oral administration includes tablets, pills, powders, granules, capsules, etc., and this solid preparation is prepared by mixing at least one excipient, for example, starch, calcium carbonate, sucrose, lactose and gelatin. In addition to simple excipients, lubricants such as magnesium stearate and talc are also used. In addition, the liquid formulations for oral administration include suspensions, internal solutions, emulsions, syrups, etc. In addition to water and liquid paraffin, which are commonly used simple diluents, various excipients, for example, wetting agents, sweeteners, fragrances, preservatives, etc. may be included.

The formulation for parenteral administration includes sterile aqueous solutions, non-aqueous solutions, suspensions, emulsions, lyophilized formulations, and suppositories. Non-aqueous solvents and suspensions may include propylene glycol, polyethylene glycol, vegetable oils such as olive oil, and injectable esters such as ethyl oleate. As the base of the suppository, witepsol, macrogol, tween 61, cacao butter, laurin, glycerogelatin, etc. may be used. The parenteral administration may be performed using an injection method such as an external skin or intraperitoneal injection, rectal injection, subcutaneous injection, intravenous injection, intramuscular injection, or intrathoracic injection.

The dosage of the pharmaceutical composition of the present invention varies depending on the patient's weight, age, sex, health status, diet, administration time, administration method, excretion rate and severity of disease, and may be administered once a day or in several divided doses.

The pharmaceutical composition of the present invention may be administered to an individual by various routes.

The pharmaceutical composition of the present invention may be used alone or in combination with methods using surgery, radiation therapy, hormone therapy, chemotherapy, and biological response modifiers for the prevention or treatment of diseases caused by muscle loss.

According to another aspect of the present invention, the present invention provides a pharmaceutical composition for preventing or treating diseases caused by muscle loss, the composition including a PHF20 gene expression inhibitor or a PHF20 protein activity inhibitor; or a YY1 promoter activity inhibitor.

In a specific embodiment of the present invention, the YY1 promoter activity inhibitor may be siRNA, and is preferably shown by the nucleotide sequence represented by SEQ ID NO: 2.

According to another aspect of the present invention, the present invention provides a composition for increasing muscle or inhibiting muscle loss, the composition including a PHF20 gene expression inhibitor or a PHF20 protein activity inhibitor as an active ingredient.

The composition is preferably a health functional food composition or a feed additive composition, but is not limited thereto.

In the present invention, a feed additive refers to a substance added in a trace amount to a feed for nutritional or specific purposes.

When the composition for increasing muscle or inhibiting muscle loss is a health functional food composition, the PHF20 gene expression inhibitor or the PHF20 protein activity inhibitor may be added as it is, or it may be used together with other foods or food ingredients and may be used appropriately according to a conventional method. The mixed amount of the active ingredient may be appropriately determined according to the purpose of use (prevention, health or therapeutic treatment). In general, in the production of food or beverage, the PHF20 gene expression inhibitor or PHF20 protein activity inhibitor of the present invention is added in an amount of 15% by weight or less, preferably 10% by weight or less, based on the total composition. However, in the case of long-term ingestion for health and hygiene purposes or for health control, it may be less than the above range. Since there is no problem in terms of safety, the active ingredient may be used in an amount equal to or more than the range.

There is no particular limitation on the type of the health functional food. Examples of the health functional food include candies, snacks, gums, beverages, tea, drinks, alcoholic beverages and vitamin complexes, and include all health functional foods in a conventional meaning.

When the composition for increasing muscle or inhibiting muscle loss is a feed additive composition, the feed additive composition of the present invention is provided as a feed by appropriately mixing it with a feed raw material, and the feed raw materials include grains, crude rice bran, vegetable oil meals, animal feed raw materials, other feed raw materials, and refined products, etc., but are not limited thereto.

### [Modes of the Invention]

Hereinafter, the present invention will be described in more detail through examples. These examples are only for illustrating the present invention, and it will be apparent to those of ordinary skill in the art that the scope of the present invention is not to be construed as being limited by these examples.

### Experimental material

Anti-PHF20 antibody was purchased from Cell Signaling Technology (Beverly, MA). Anti-β actin antibody was obtained from Sigma Aldrich (St. Louis, USA). Anti-YYl, anti-MyoD and anti-myosin heavy chain antibodies were purchased from Santa Cruz Biotechnology (Santa Cruz, CA). Anti-myosin (MF20) antibody was obtained from DSHB, University of Iowa. HRP-conjugated anti-mouse, anti-rabbit and anti-goat IgG antibodies were obtained from Koma biotech (Seoul, Korea). pYY-luciferase was purchased from Panomics (California, USA). A luciferase assay system kit was purchased from Promega (Madison, USA). Tet-on advanced inducible gene expression system kit was obtained from Clontech (Takara Bio Company, USA).

### Experimental Example 1. Cell culture

### 1-1. C₂C₁₂ cell culture and differentiation

C2C12 cells, which are premyocytes, were cultured at 37°C and 5% CO₂ conditions using DMEM medium. The DMEM (WELGENE, Gyeongsan, Korea) medium contains 10% FBS (fetal bovine serum) (HyClone, Logan, USA) and 1% Antibiotic-Antimycotic (Gibco, Detroit, USA).

For cell differentiation, the medium was replaced with a differentiation medium (DM) and then the culture was performed, and the medium was replaced every two days. The differentiation medium contains 2% horse serum and 1% Antibiotic-Antimycotic (Gibco, Detroit, USA). The cell differentiation was initiated 24 hours after the medium change.

### 1-2. C₂C₁₂/Tet-On inducible PHF20 cells

C₂C₁₂/Tet-On inducible PHF20 cells were prepared using C2C12 cells. Specifically, in order to prepare C₂C₁₂-TET3G myoblasts, C₂C₁₂ cells were treated with lentivirus-TET3G, and C₂C₁₂-TET3G myoblasts were obtained by selecting with G418 (1.2 mg/ml). The C₂C₁₂-TET3G cells were transiently transfected with pLVX-TRE3G-PHF20. Tet-On-inducible PHF20 cells were selected among the transiently transfected cells using puromycin (3 µg/ml). When the selected cells (C₂C₁₂/Tet-On-inducible PHF20 cells) are cultured in a medium containing doxycycline (Doxy), the expression of PHF20 is inducible.

In the following experiment, in order to induce the expression of PHF20 in C2C12 cells, it was cultured in a medium containing doxycycline at a concentration of 100 ng/ml.

### Experimental Example 2. Western blotting

The prepared cells were placed on ice, and the cells were treated with a lysis buffer to prepare a cell lysate. The lysis buffer includes 50 mM Tris-HCl(pH 7.5), 1% v/v Nonidet P-40, 120 mM NaCl, 25 mM sodium fluoride, 40 mM βphosphate, 0.1 mM sodium orthovanadate, 1 mM phenylmethylsulfonyl fluoride, 1 mM benzamidine and 2 µM microcystin-LR. Cell lysates were centrifuged at 13,000 rpm for 30 minutes to obtain proteins. The protein was developed by SDS-PAGE using a 10.0% to 7.5% gel. The developed protein was transferred to an Immobilon-P membrane (Millipore). Thereafter, the membrane was blocked for 1 hour by treating with 1X TBS (tri-buffered saline buffer; 140 mM NaCl, 2.7 mM KCl, 250 mM Tris HCl, pH 7.4) containing 5% skim milk and 0.2% Tween-20. Then, it was treated with the diluted 1000-fold primary antibody for each protein and cultured overnight at 4°C. After the reaction, the membrane was washed with TBS. The washed membrane was treated with a 2000-fold diluted secondary antibody (horseradish peroxidase-conjugated anti-mouse IgG or anti-rabbit IgG (Komabiotech, Seoul, Korea)) and then cultured. After culture, the membrane was washed with TBS, and protein was detected according to the manufacturer's manual.

### Experimental Example 3. Real-time quantitative reverse transcription-polymerase chain reaction (qRT-PCR)

C₂C₁₂/Tet-On inducible PHF20 cells were seeded in 6-well plates and cultured for 24 hours. In order to induce the expression of PHF20, the C₂C₁₂/Tet-On inducible PHF20 cells were treated with doxycycline. The day after treatment with doxycycline, total RNA was extracted from the cells using a QuickGene RNA kit (Fujifilm's Life Science System, Tokyo, Japan). In addition, cDNA was synthesized from the extracted total RNA using SuPrimeScript RT Premix (GeNet Bio, Cheonan, Korea). The amplification of cDNA was performed with Prime Q-Mastermix (GeNet Bio, Cheonan, Korea) and CFX96T Real-time System (Bio-Rad, Hercules, CA, USA). Through qRT-PCR analysis of each cDNA, duplex DNA formation was measured using SYBR green dye and the StepOne Plus real-time PCR system (Invitrogen, Carlsbad, CA).

The primers used in this experiment are shown in Table 1.

**[Table 1]**

| | Primer | Sequence (5'-3') |
|---|---|---|
| mouse GAPDH forward (SEQ ID NO: 4) | | TCCAGTATGACTCCACTC |
| mouse GAPDH reverse (SEQ ID NO: 5) | | ATTTCTCGTGGTTCACAC |
| mouse PHF20 forward (SEQ ID NO: 6) | | CATTGACTACGAAGAAGGGAG |
| mouse PHF20 reverse (SEQ ID NO: 7) | | CTTCTCTAAAGGGCGCAGATA |
| mouse YY1 forward (SEQ ID NO: 8) | | CAGAAGCAGGTGCAGATCAGA CCCT |
| mouse YY1 reverse (SEQ ID NO: 9) | | GCACCACCACCCACGGAATCG |
| mouse MyoD forward (SEQ ID NO: 10) | | TACAGTGGCGACTCAGATGC |
| mouse MyoD reverse (SEQ ID NO: 11) | | GAGATGCGCTCCACTATGCT |

### Experimental Example 4. Detection of myosin heavy chain (MHC) through immunochemical staining analysis

C₂C₁₂/Tet-On inducible PHF20 cells were seeded in a 12-well plate containing sterile coverslips at a concentration of 1 × 10⁵ cells/well and cultured at 37°C and 5% CO₂ conditions. The cells of the doxycycline untreated group (-Doxy) were cultured for 24 hours without treatment with doxycycline, and the doxycycline-treated group (+Doxy) was cultured for 24 hours after replacing the medium with doxycycline-containing medium. After replacing the medium of each cultured cell with a differentiation medium, the cells were cultured for 1 day (D1) or 3 days (D3) to allow differentiation. The cells were then washed with PBS at 37°C. The washed cells were cultured in 4% paraformaldehyde for 15 minutes, fixed on coverslips, and washed twice with PBS. For blocking, coverslips were treated with 1% bovine serum albumin (BSA), and then cultured with shaking at room temperature for 1 hour. In addition, an anti-myosin (MF-20) antibody was diluted at a ratio of 1 : 200 and added to the cells, and the mixture was cultured with shaking at 4 °C overnight. After shaking culture, the coverslips were washed three times with PBS. FITC secondary antibody (1 : 1,000) (in 5% skim milk) was added and then cultured with shaking for 6 hours in the dark at room temperature. After shaking culture, the coverslips were washed three times with PBS. After replacing the medium of the washed coverslip with a mounting medium containing DAPI VECTASHIELD (St. Louis, USA), it was bound to the slide and analyzed for fluorescence.

### Experimental Example 5. Luciferase reporter gene analysis

C₂C₁₂/Tet-On inducible PHF20 cells were seeded in 6-well plates at a concentration of 3 × 10⁵ cells/ml. The seeded cells were transfected with 1 µg of plasmid containing YY1-luciferase according to the manufacturer's manual. Transfected cells were used for experiments after 24 hours.

Transfected cells were treated with reporter lysis buffer to lyse the cells. The reporter lysis buffer includes 25 mM Tris-phosphate, 2 mM DTT, 2 mM trans-1,2-cyclohexanediamine-N,N,N',N'-tetraacetic acid, 10% (v/v) glycerol and 1% (v/v) Triton x-100. The luciferase assay substrate (Promega) was added to 20 µg of cell lysates, and they were cultured. For luciferase activity, luminescence was measured for 10 seconds using a luminometer (Duo Lumat LB 9507).

### Experimental Example 6. Chromatin-immunoprecipitation (ChiP) analysis

Chromatin immunoprecipitation analysis was performed according to the manufacturer's manual (Upstate Biotechnology, Lake Placid, NY, USA). Specifically, C₂C₁₂ cells were differentiated for 3 days and was treated with formaldehyde for 7.5 minutes. Thereafter, the cells were washed twice with PBS containing a protease inhibitor and then transferred onto the ice. Cells on ice were treated with SDS-lysis buffer and cultured for 10 minutes, and the lysate was collected. The lysis buffer includes 50 mM Tris-HCl (pH 8.0), 1% SDS, 10 mM EDTA, 1 mM PMSF, 1 µg/ml aprotinin and 2 µg/ml pepstatin. The collected lysates were cultured overnight at 4°C after treatment with primary antibody or control IgG. Thereafter, 30 µl of protein A beads (containing 500 µg/ml BSA and 200 µg/ml salmon sperm DNA) were cultured together at 4°C for 3 hours in the culture. After culture, the DNA sample was purified using QIA quick PCR purification kit (Qiagen, Hilden, Germany) and quantified by qRT-PCT. qRT-PCT results were expressed as a percentage of the entered value. The sequence of mouse YY1 (-200/-55), a promoter-specific primer used in this experiment, is as follows.
- mouse YY1(-200/-55): 5'-CGCTGCCTTCCTCCCTCT-3'(forward, SEQ ID NO: 12), 5'-CGTCCGTGGCGATGTAGA-3'(reverse, SEQ ID NO: 13)

### Experimental Example 7. Preparation of PHF20 transgenic mice

In order to prepare PHF20 transgenic mice, cDNA encoding human PHF20 was subcloned downstream of the CMV promoter of pcDNA3 vector, and WT C57BL/6 mice were transformed with the vector. As a control group, WT C57BL/6 mice were used and denoted as WT. Mouse PHF20-TG and WT were reared in an environment of 12h/12h long cycle, 50% to 60% humidity, and 22°C ambient temperature. In addition, all animal experiments were performed in animal facilities in accordance with institutional guidelines. The experimental protocol was approved by the Chungnam National University (CNU-00890) review committee.

### Experimental Example 8. Histological analysis and immunochemical staining analysis

Muscles collected from mice were treated with 10% formalin and then cultured overnight at 4°C and fixed. The fixed muscle samples were embedded in paraffin blocks. A 5 µm-thick section was prepared by cutting the paraffin block. Slices from adjacent sites were additionally prepared, and slides were prepared sequentially.

For general morphological analysis, the prepared slides were stained with hematoxylin and eosin (H&E).

In addition, for immunochemical staining analysis, the slices on the slide were deparaffinized with xylene and rehydrated with ethanol. Thereafter, the slides were immersed in a peroxidase quenching solution and reacted for 10 minutes. After the reaction, the slides were washed twice with PBS for 5 minutes each, and 2 drops of reagent A were added to the slide for blocking and then cultured for 30 minutes. After culture, the slides were washed twice with PBS. The washed slides were treated with a primary antibody (anti-MHC) and cultured overnight at 4°C. The cultured slides were washed with PBS and then treated with reagent B, a biotinylated secondary antibody and cultured at room temperature for 1 hour. After the cultured slides were washed with PBS, enzyme-conjugated reagent C was dropped on the slides and washed with PBS. After washing with PBS, DAB chromophore; and a mixture of reagents D1, D2 and D3; were dropped on the slide. The signal was observed under a fluorescence microscope. After stopping the reaction with distilled water, slide images were taken with a fluorescence microscope.

### Experimental Example 9. Statistical analysis

Data are expressed as mean ± S.E. Differences between groups in at least 3 experiments were analyzed using Student's t-test, and P < 0.05 was considered statistically significant. Image J software (version 1.47) was also used for quantitative analysis of the results.

### Example 1. Analysis of expression of YY1 and PHF20 during myogenic differentiation

In order to evaluate the changes in PHF20 (PHD finger protein 20) in myogenic differentiation, YY1 (Yin Yang 1) and PHF20 mRNA and protein expression in myogenic differentiation were analyzed by qRT-PCR and Western blotting.

### 1-1. PHF20

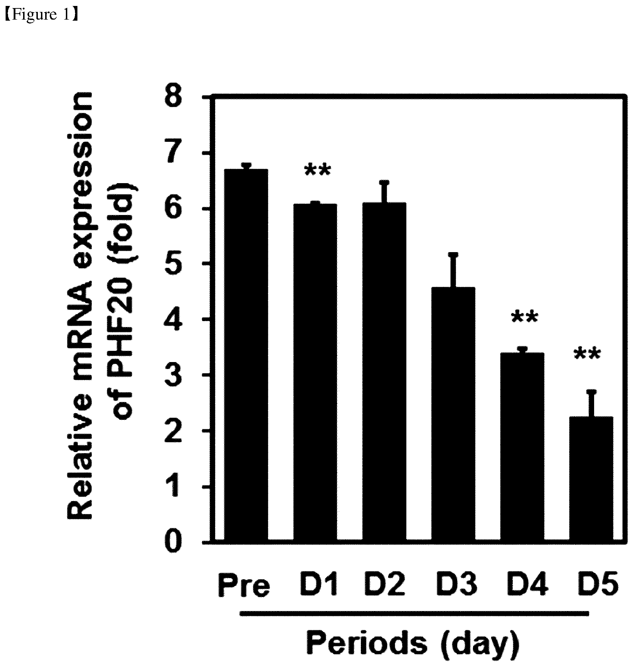

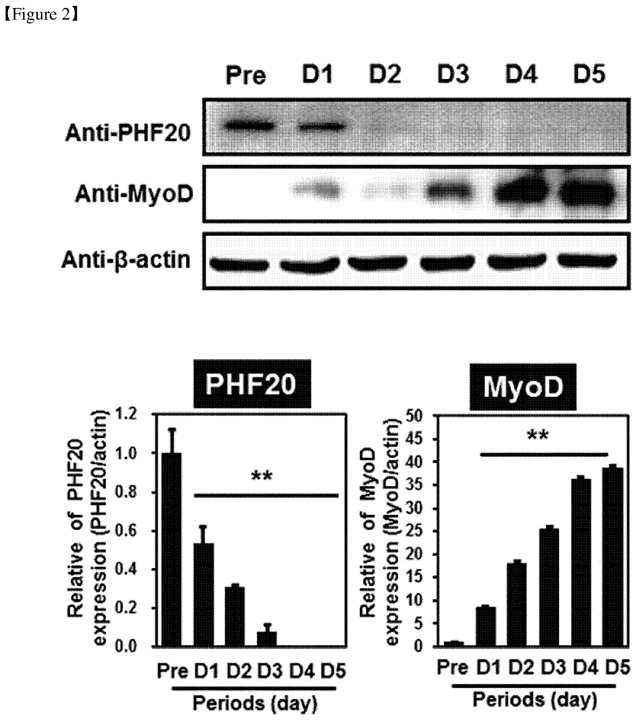

24 hours after C2C12 cells were seeded, myogenic differentiation was inducible by replacement with differentiation medium (DM) containing 2% horse serum. PHF20 mRNA expression of cells was measured daily during myogenic differentiation by qRT-PCR. In addition, the protein expression of PHF20 and myoD was checked daily during the myogenic differentiation through western blotting. The results of analyzing PHF20 mRNA and protein expression are shown in FIGS. 1 and 2, respectively.

As shown in FIGS. 1 and 2, it was confirmed that the expression of PHF20 mRNA and protein decreased as myogenic differentiation progressed. Meanwhile, it was confirmed that MyoD protein expression increased as myogenic differentiation progressed. These results suggest that PHF20, a transcription factor, may be involved in myogenic differentiation in C₂C₁₂ cells.

### 1-2. YY1

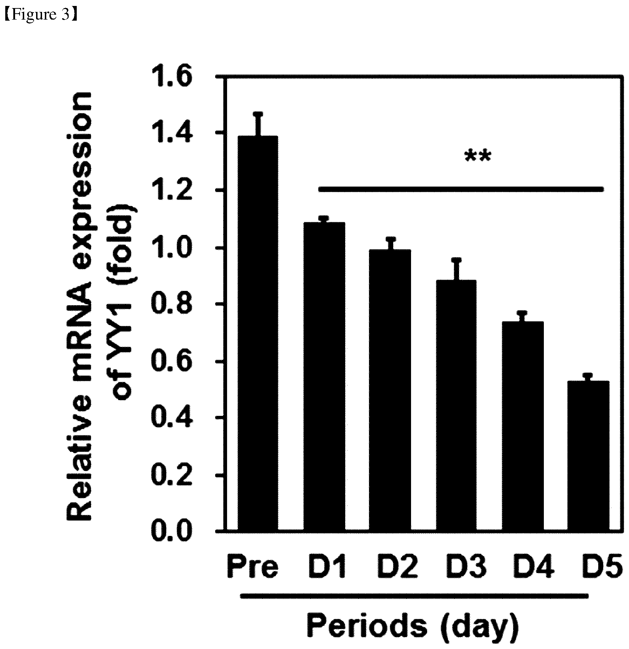

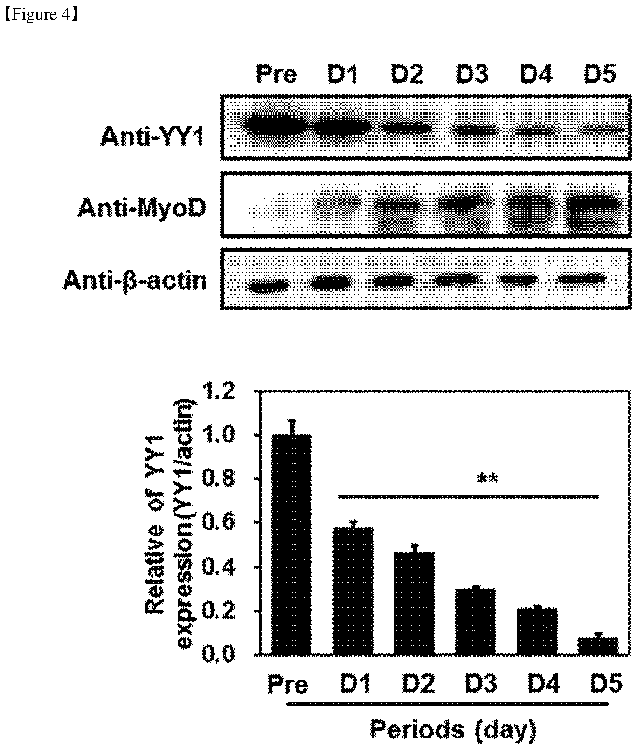

It was confirmed that YY1 expression is increased in cells overexpressing PHF20 through previous studies. Accordingly, mRNA and protein expression of YY1 were analyzed in the same manner as in Example 1-1. The results of analyzing the mRNA and protein expression of YY1 are shown in FIGS. 3 and 4, respectively.

As shown in FIGS. 3 and 4, it was confirmed that mRNA expression of YY1 decreased as myogenic differentiation progressed. In addition, it was confirmed that as myogenic differentiation progressed, the protein expression of MyoD increased, but the protein expression of YY1 decreased.

### Example 2. Evaluation of the effect of PHF20 expression on the differentiation of C₂C₁₂ cells

The effect of PHF20 expression on myogenic differentiation was further evaluated.

### 2-1. Effect of PHF20 expression on myoD protein expression

After preparing C2C12 cells (Pre), they were divided into a doxycycline untreated group (-Doxy) and a treated group (+Doxy). Thereafter, the doxycycline-untreated group (-Doxy) was cultured for 24 hours without treatment with doxycycline on the prepared cells, and the doxycycline-treated group (+Doxy) was cultured for 24 hours after replacing with a medium containing doxycycline. C2C12 cells untreated or treated with doxycycline were used in the experiment and marked as Pre.

In order to prepare differentiated myofibers (D1, D3, and D5), the medium of C2C12 cells untreated or treated with doxycycline was replaced with a differentiation medium and then cultured. The cultured cells were designated as D1, D3, and D5, respectively, according to the number of days of differentiation.

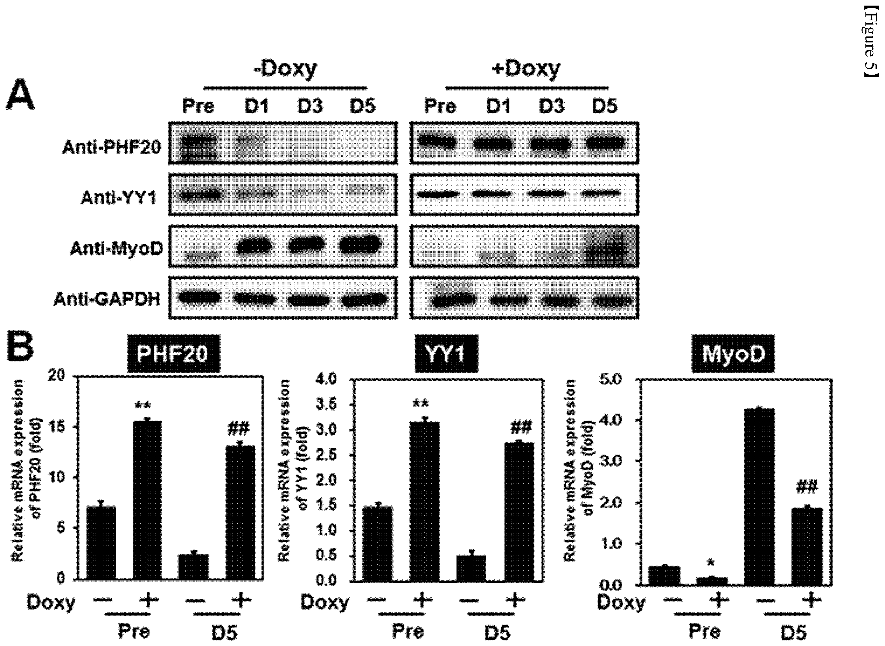

Each cell lysate was prepared by treating the prepared cells with a lysis buffer, and protein expression of PHF20, YY1 and MyoD was analyzed by Western blotting. The results of analyzing the protein expression of PHF20, YY1 and MyoD are shown in FIG. 5A.

As shown in FIG. 5A, in the control group untreated with doxycycline (left panel), the protein expression of PHF20 and YY1 decreased, and the expression of MyoD showed a tendency to increase during myogenic differentiation. On the other hand, it was confirmed that in the doxycycline-treated group (right panel), the expression of PHF20 was inducible, and the expression of MyoD was lower than that of the control group.

### 2-2. Effect of PHF20 expression on mRNA expression of myoD

After extracting total RNA from C2C12 cells (Pre) and differentiated myofibers (D5) treated with doxycycline (+Doxy) or untreated (-Doxy) of Example 2-1, mRNA expression of PHF20, YY1 and MyoD were analyzed by qRT-PCR. The result of analyzing the mRNA expression is shown in FIG. 5B.

As shown in FIG. 5B, it was confirmed that the expression of PHF20, YY1 and MyoD during muscle differentiation had the same tendency as protein expression, that is, the cells in which PHF20 expression was induced had reduced MyoD expression.

### 2-3. Effect of PHF20 expression on myotube formation

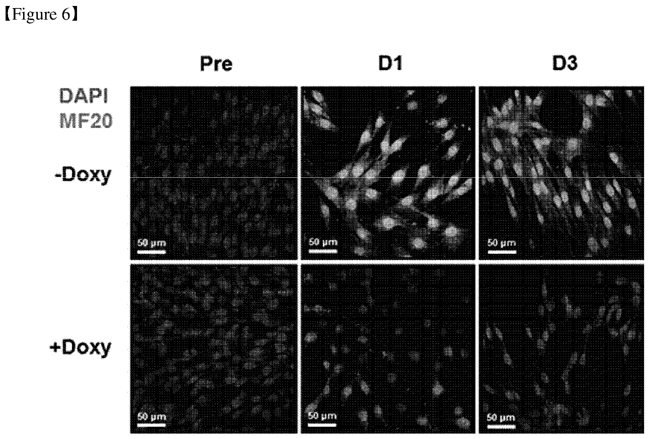

In order to confirm the effect of PHF20 on myotube formation, the expression of myosin heavy chain was investigated through immunochemical staining analysis. Specifically, doxycycline-treated (+Doxy) or untreated (-Doxy) C2C12 cells (Pre) and differentiated myofibers (D1 and D3) were prepared. The prepared cells were used to perform immunochemical staining analysis using an anti-MF antibody (green). The myosin heavy chain was stained with an anti-MF antibody and displayed in green, and the cell nucleus was stained with 4',6-diamidino-2-phenylindole (DAPI) and displayed in blue. The results of the immunochemical staining analysis are shown in FIG. 6.

As shown in FIG. 6, it was confirmed that myotube formation in cells in which PHF20 expression was induced by doxycycline (+Doxy, D1 and D3) was inhibited during muscle differentiation. However, it was confirmed that C₂C₁₂ cells were differentiated into myotubes in the control group untreated with doxycycline (-Doxy, D1 and D3).

### 2-4. Effect of inhibition of PHF20 expression on MyoD expression

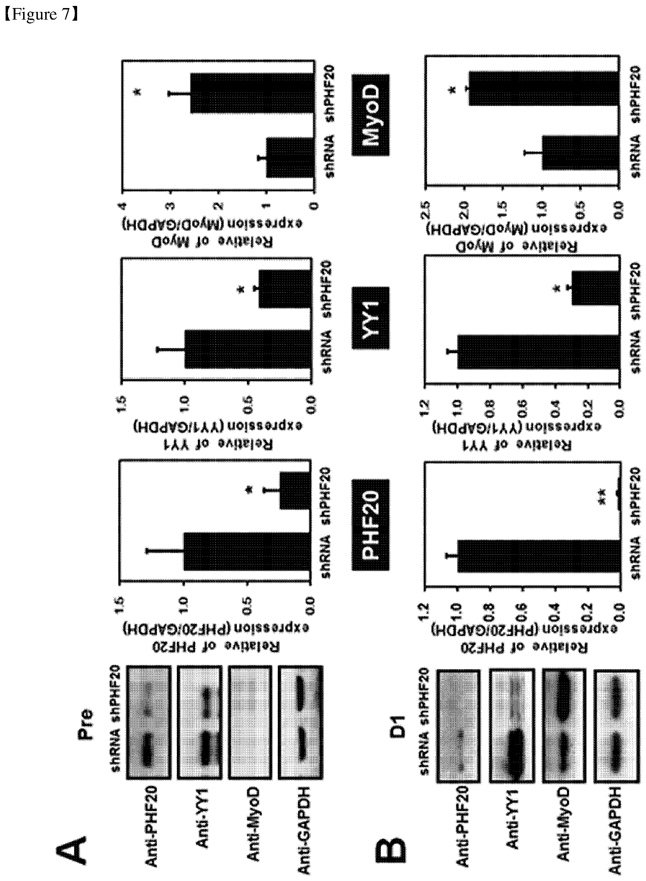

The effect of inhibition of PHF20 expression on myoD expression was confirmed. For this, C₂C₁₂ cells (Pre) and differentiated myofibers (D1) were prepared. The prepared cells were transfected with a lentivirus expressing shRNA (shPHF20) for the PHF20 sequence shown by the nucleotide sequence represented by SEQ ID NO: 1. shRNA for the scrambled sequence shown by the nucleotide sequence represented by SEQ ID NO: 3 was used as a control group. Transfected cells were cultured for 24 hours. The cells were lysed after culture, and the cell lysate was used to perform Western blotting. The results of pre-differentiated C2C12 cells (Pre) are shown in FIG. 7A, and the results of differentiated myofibers (D1) are shown in FIG. 7B.

As shown in FIG. 7, in pre-differentiated C₂C₁₂ cells (Pre), inhibition of PHF20 expression by shRNA induced inhibition of YY1 expression and increased MyoD expression. It was confirmed that the above phenomenon was more pronounced in the myofiber (D1), which is the first day of differentiation.

### Example 3. Effect of PHF20 expression on YY1 promoter expression

Through Examples 1 and 2, it was confirmed that the increase in PHF20 expression during myogenic differentiation increased the YY1 expression and decreased the MyoD expression. Accordingly, the effect of PHF20 expression on muscle differentiation was additionally evaluated through YY1 promoter analysis.

### 3-1. Evaluation of PHF20 expression and YY1 promoter expression according to the treatment concentration of doxycycline

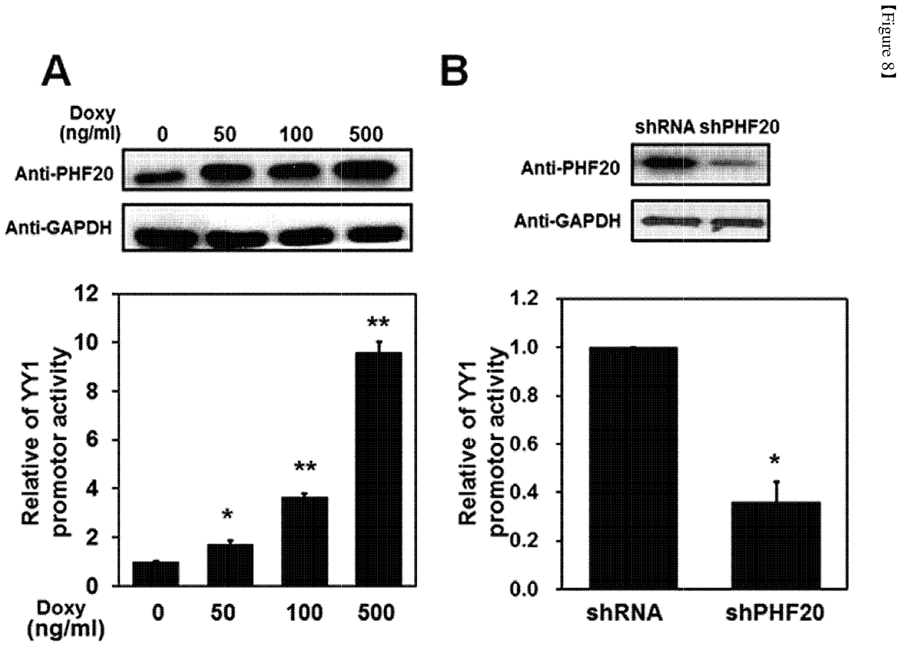

The expression of PHF20 and YY1 promoters according to the concentration of doxycycline was examined. C2C12 cells (Pre) were transfected with the YY1-luciferase gene. The transfected cells were treated with doxycycline at different concentrations (0, 50, 100 and 500 ng/ml) and then cultured for 24 hours. After culture, Western blotting and luciferase reporter gene analysis were performed for the C2C12 cells. The Western blotting is to confirm the expression of PHF20, and the luciferase reporter gene analysis is to confirm the activity of the YY1 promoter. The results of Western blotting and analysis of the luciferase reporter gene are shown in FIG. 8A.

As shown in FIG. 8A, it was confirmed that doxycycline induces the PHF20 expression, and the PHF20 expression was dependent on the concentration of doxycycline. In addition, it was confirmed that the activity of the YY1 promoter depends on the expression level of PHF20.

### 3-2. Evaluation of PHF20 and YY1 promoter expression according to shRNA transfection

The expression of PHF20 and YY1 promoters following shRNA transfection was examined. Specifically, C2C12 cells (Pre) were prepared, and the prepared cells were transfected with a lentivirus expressing shRNA for a scrambled sequence (shRNA) or a PHF20 sequence (shPHF20). After the transfected cells were cultured for 24 hours, the cells were further transfected with the YY1-luciferase gene. Thereafter, the cells were lysed, and Western blotting and luciferase reporter gene analysis were performed using the cell lysate, and the results are shown in FIG. 8B.

As shown in FIG. 8B, it was confirmed that the decrease in PHF20 expression caused by shRNA inhibited the activity of the YY1 promoter. This means that PHF20 positively regulates YY1 expression in premyocytes at the transcript level.

### 3-3. Evaluation of expression of PHF20 and YY1 promoters according to the period of myogenic differentiation

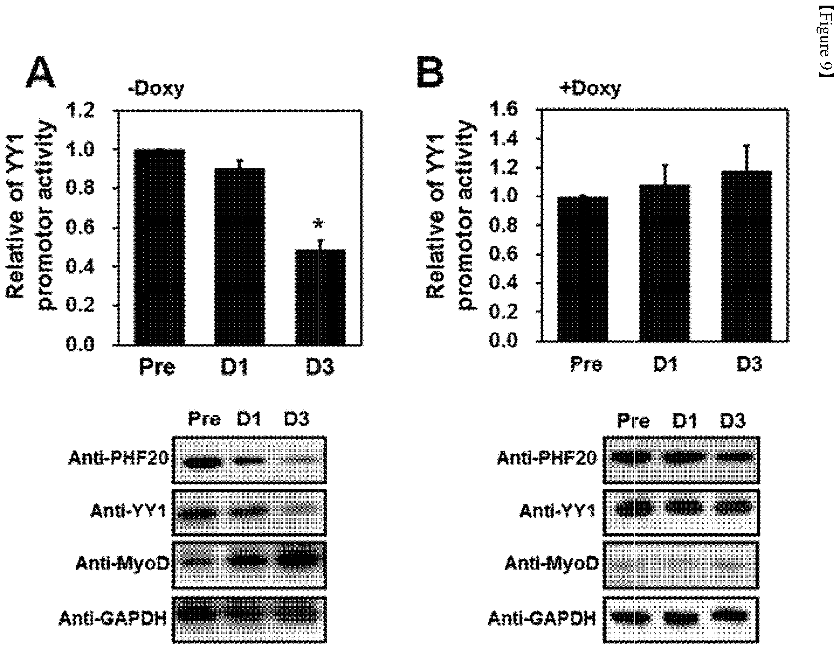

The effect of inhibition of PHF20 expression on YY1 promoter expression according to the myogenic differentiation period was analyzed. For this, YY1 promoter analysis was performed using C2C12 cells, which are premyocytes. Specifically, C2C12 cells (Pre) not treated with doxycycline were differentiated by culturing in a differentiation medium, and the cells were transfected with the YY1-luciferase gene at each differentiation time point (D1 and D3). Expressions of PHF2, YY1 and MyoD in the transfected cells were analyzed by Western blotting. In addition, the activity of the YY1 promoter was confirmed through luciferase reporter gene analysis. C2C12 cells (Pre) treated with doxycycline were tested in the same manner as above. The experimental results using the C2C12 cells not treated with doxycycline are shown in FIG. 9A, and the experimental results using the C₂C₁₂ cells treated with doxycycline are shown in FIG. 9B.

As shown in FIG. 9A, as the myogenic differentiation of C₂C₁₂ cells progressed, the YY1 promoter activity decreased so that the expression of PHF20 and YY1 decreased, and the expression of MyoD increased.

As shown in FIG. 9B, it was confirmed that PHF20 expression induced by doxycycline induced YY1 promoter activity during myogenic differentiation, and thus MyoD expression was reduced.

These results indicate that the expression level of PHF20 is important for the regulation of myogenic differentiation through YY1 and YY1 promoters.

### 3-4. Confirmation of regulation method of YY1 promoter by PHF20

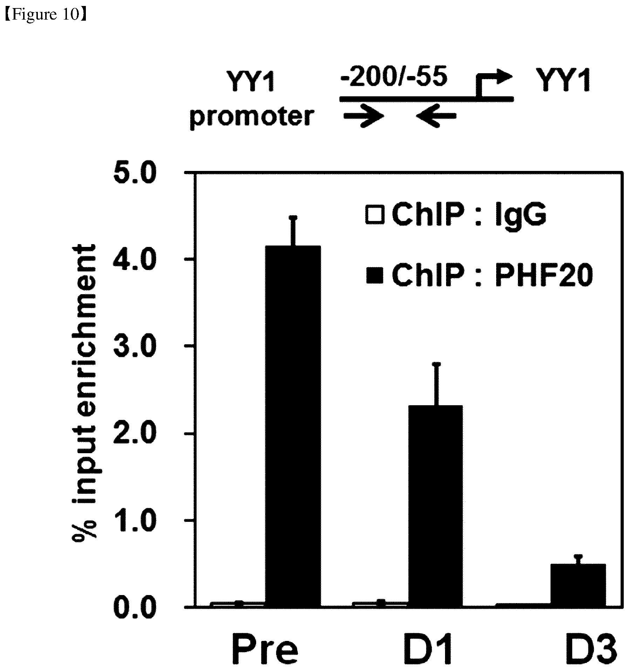

It was confirmed how PHF20 regulates the YY1 promoter through chromatin-immunoprecipitation (ChiP) analysis. For the chromatin immunoprecipitation analysis, C2C12 cells (Pre) and differentiated myofibers (D1 and D3) were used, and primer mouse YY1 (-200/-55) was used. IgG was used as a control group. The results of chromatin immunoprecipitation analysis are shown in FIG. 10.

As shown in FIG. 10, the binding of PHF20 to the YY1 promoter (-200/-55) in C₂C₁₂ cells was reduced during myogenic differentiation. These results suggest that PHF20 is a transcription factor for regulating myogenic differentiation and directly binds to the YY1 promoter.

### Example 4. Effect of YY1 expression on MyoD expression

Through Example 3, it was confirmed that PHF20 directly regulates YY1. Accordingly, the effect of the YY1 promoter expression on the downstream gene MyoD was examined in this example.

### 4-1. Western blotting

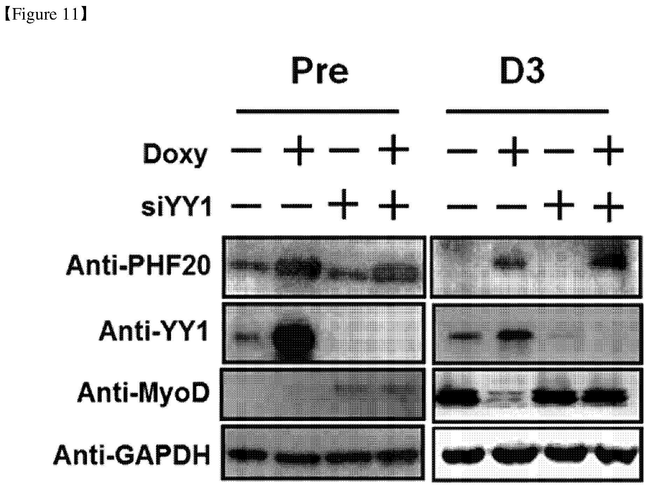

In order to examine the effect of expression of the YY1 promoter on the downstream gene MyoD, C2C12 cells (Pre) treated with doxycycline (100 ng/ml) (+Doxy) or untreated (-Doxy) were prepared. The C2C12 cells were cultured in a differentiation medium to differentiate them into muscle. On the third day of differentiation (D3), the cells were transfected with siRNA or siYY1 (SEQ ID NO: 2) and cultured for 24 hours. After culture, cell lysates were prepared. The expression of PHF20, YY1 and MyoD was analyzed by Western blotting. As a control group, C2C12 cells (Pre) treated with doxycycline (100 ng/ml) (+Doxy) or untreated (-Doxy) were used. Western blotting results are shown in FIG. 11.

As shown in FIG. 11, overexpression of PHF20 induced a decrease in MyoD expression in C2C12 cells (Pre), and on the third day of differentiation (D3), myogenic differentiation was induced as well as YY1 expression was induced in pre-myoblasts (first 2 lanes for each condition). In addition, inhibition of YY1 expression by shRNA increased MyoD expression (last two lanes for each condition).

### 4-2. Immunochemical staining analysis

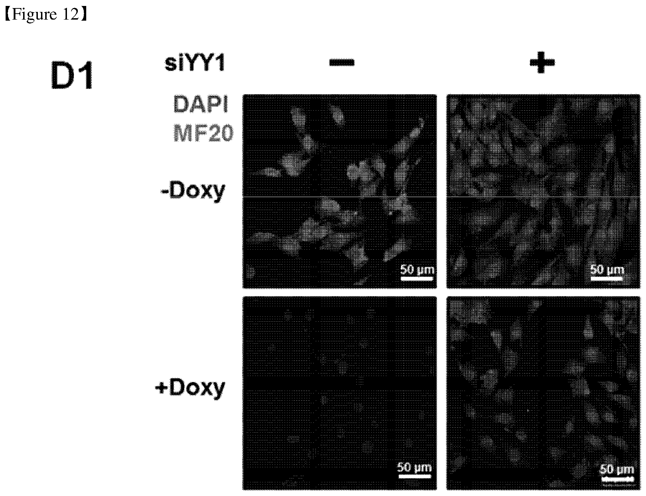

In order to confirm the effect of YY1 on myotube formation, the expression of myosin heavy chain was examined through immunochemical staining analysis. Specifically, differentiated myofibers (D1) treated with doxycycline (+Doxy) or untreated (-Doxy) were prepared. The prepared cells were transfected with siRNA or siYY1 and cultured for 24 hours. The cultured cells were used to perform immunochemical staining analysis using an anti-myosin (MF20) antibody. Myosin heavy chain was stained with an anti-myosin (MF20) antibody and displayed in green, and cell nuclei were stained with 4',6-diamidino-2-phenylindole (DAPI) and displayed in blue. The results of the immunochemical staining analysis are shown in FIG. 12.

As shown in FIG. 12, it was confirmed that reduction in expression of YY1 caused by shRNA promoted myotube formation (+siYY1, -Doxy). On the other hand, it was confirmed that the expression of PHF20 induced by doxycycline inhibited myotube formation (-siYY1, +Doxy). In particular, it was confirmed that differentiation was restored when the expression of YY1 was inhibited despite the overexpression of PHF20 (+siYY1, +Doxy). These results suggest that PHF20 regulates differentiation regulators such as MyoD through YY1.

### Example 5. Evaluation of the effect of PHF20 expression on myogenic differentiation in PHF20 transgenic mice

### 5-1. PHF20, YY1 and MHC expression analysis and confirmation of phenotype in PHF20 transgenic mice

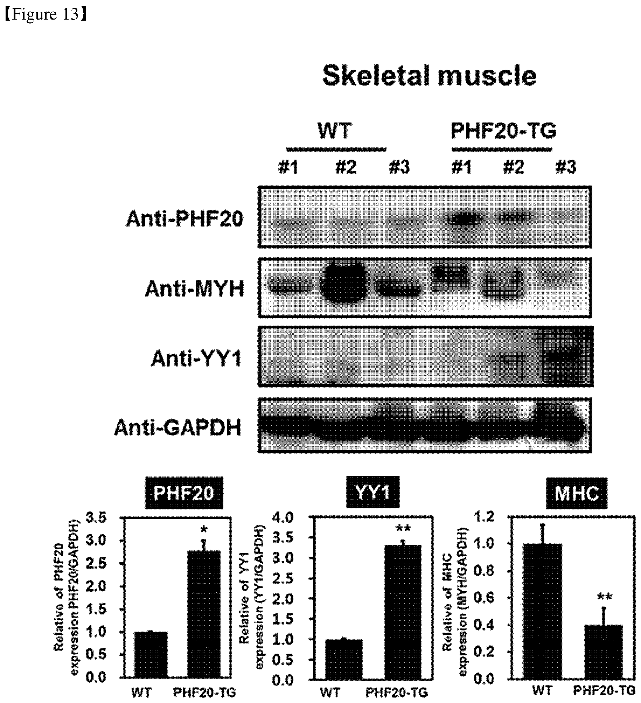

Skeletal muscle tissue was isolated from PHF20 transgenic mice. The isolated skeletal muscle tissue was lysed to prepare cell lysates, and the expression of PHF20, YY1 and myosin heavy chain (MHC) was analyzed through Western blotting. The myosin heavy chain is a myogenic differentiation marker, and its expression increases as differentiation progresses. 5-month-old male WT mice were used as a control group. Western blotting results are shown in FIG. 13.

As shown in FIG. 13, the expression of PHF20 and YY1 was significantly increased in the PHF20 transgenic mice compared to the control group (WT). However, it was confirmed that the expression of the myosin heavy chain (MHC), a marker of myogenic differentiation, was decreased in the PHF20 transgenic mice compared to the control group.

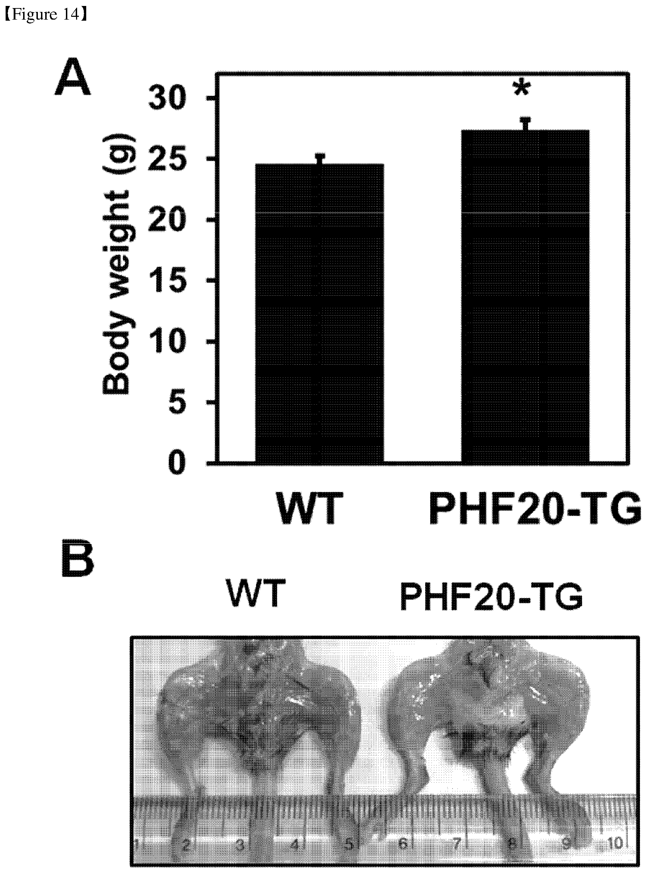

PHF20 transgenic mice were weighed before sacrifice for the next experiment. Thereafter, the PHF transgenic mice were dissected and the thigh muscles and muscles were observed. The weight measurement results of the PHF20 transgenic mice are shown in FIG. 14A, and the results of observing the thigh muscle are shown in FIG. 14B.

As shown in FIG. 14A, it was confirmed that the PHF20 transgenic mice significantly increased their body weight compared to the control group (WT).

As shown in FIG. 14B, it was confirmed that the muscle of the dissected PHF20 transgenic mouse had a lighter muscle color than that of the control group (WT), which means that it had less muscle and more fat accumulation compared to the control group.

The above results indicate that the PHF20 transgenic mice gained weight due to fat accumulation.

### 5-2. Histological analysis and immunochemical staining analysis

A slide was prepared by slicing the gastrocnemius tissue of the PHF20 transgenic mouse (PHF20-TG) and the control group (WT) in a transverse direction (cross section) or longitudinal direction (longitudinal section).

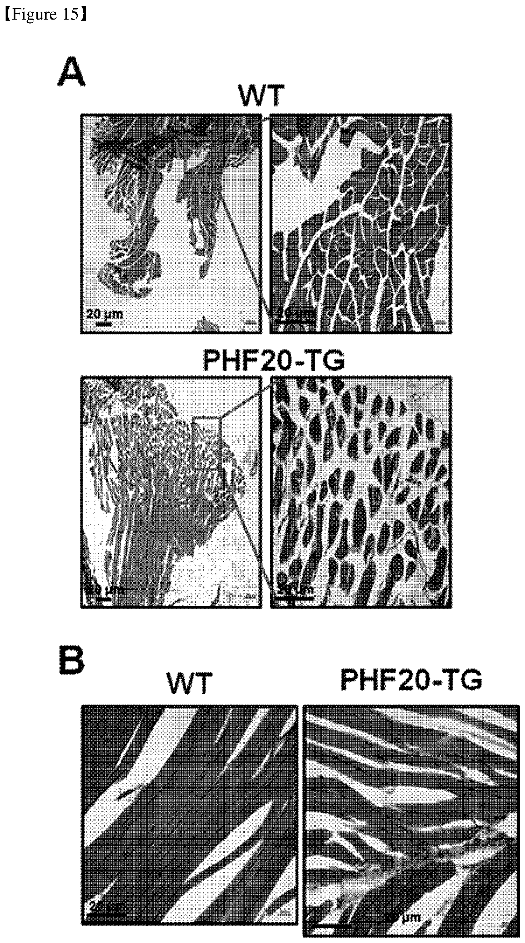

Tissues of the prepared transverse or longitudinal slides were stained with hematoxylin and eosin (H&E). The result of observing the cross-section of the gastrocnemius tissue is shown in FIG. 15A, and the result of observing the longitudinal section is shown in FIG. 15B.

As shown in FIG. 15A, it was confirmed that the PHF20 transgenic mice had fewer myofibers compared to the control group. In addition, it was confirmed that the PHF20 transgenic mice had more round myofibers and many empty spaces (white color) between the myofibers compared to the control group.

As shown in FIG. 15B, as a result of observing the longitudinal cross-section of the muscle, it was confirmed that the PHF20 transgenic mice had less myofiber compression compared to the control group, which means that the muscle integrity was low.

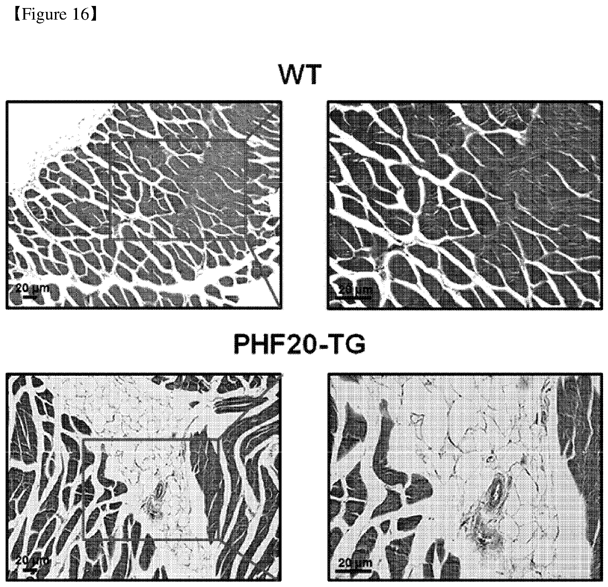

In addition, the cross-sectional slides stained with hematoxylin and eosin were additionally observed, and the results are shown in FIG. 16.

As shown in FIG. 16, it was observed that the control group (WT) had almost no adipose tissue between the muscles, whereas the PHF20 transgenic mice had adipose tissue between the muscles.

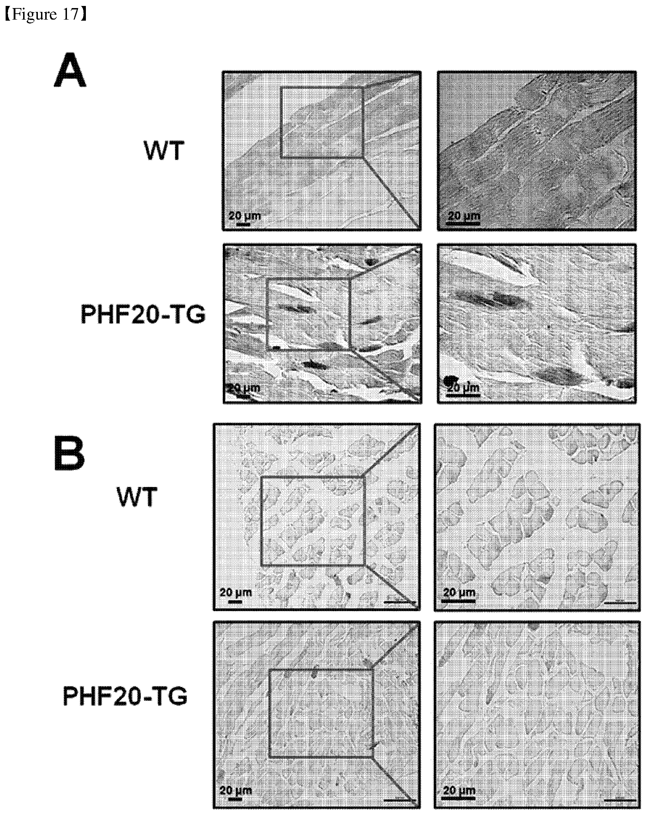

The gastrocnemius muscle tissue of the prepared transverse or longitudinal slide was observed through immunochemical staining analysis. The results of observing the longitudinal cross-section of the gastrocnemius tissue are shown in FIG. 17A, and the results of observing the cross-sections are shown in FIG. 17B.

As shown in FIG. 17A, it was confirmed that the PHF20 transgenic mice had fewer myofibers having a shorter length compared to the control group (WT). In addition, it was confirmed that the expression of MHC in the PHF20 transgenic mice was decreased compared to the control group (WT).

As shown in FIG. 17B, it was found that the PHF20 transgenic mice had voids between the myofibers compared to the control group (WT), which means that the degree of compression of the myofibers was low.

The above results confirmed that PHF20 had a negative effect on muscle development in vivo.

In sum, the above experimental results confirmed that the expression of PHF20 affects myogenic differentiation through the mechanism shown in FIG. 18. More specifically, PHF20 gene knockout, that is, inhibition of PHF20 expression causes isolation of PHF20 bound to the YY1 promoter in vitro and in vivo to increase the expression of MyoD, thereby promoting myogenic differentiation. This PHF20 inhibitor may be variously used in the field of prevention or treatment of diseases caused by muscle loss.

Hereinafter, the present invention is described in more detail through formulation examples. The formulation examples are only for illustrating the present invention, and the scope of the present invention is not to be construed as being limited by the formulation examples.

### Formulation Example 1. Preparation of pharmaceutical composition

### 1-1. Preparation of powders

20 µg of PHF20 gene expression inhibitor or PHF20 protein activity inhibitor
100 mg of lactose
10 mg of talc

The above ingredients are mixed and filled in an airtight bag to prepare powders.

### 1-2. Preparation of tablets

20 µg of PHF20 gene expression inhibitor or PHF20 protein activity inhibitor
Cornstarch 100 mg
100 mg of lactose
2 mg of magnesium stearate

The above ingredients are mixed, and then tablets are prepared by tableting according to a conventional method for preparing tablets.

### 1-3. Preparation of capsules

20 µg of PHF20 gene expression inhibitor or PHF20 protein activity inhibitor
3 mg of crystalline cellulose
14.8 mg of lactose
0.2 mg of magnesium stearate

According to a conventional method for preparing capsules, the above ingredients are mixed and filled in a gelatin capsule to prepare capsules.

### 1-4. Preparation of injections

10 µg of PHF20 gene expression inhibitor or PHF20 protein activity inhibitor
180 mg of mannitol
2974 mg of sterile distilled water for injection
26 mg disodium phosphate dihydrate

According to a conventional method for preparing injections, injections are prepared in the content of the above ingredients per 1 ampoule (2 ml).

### 1-5. Preparation of liquids

10 µg of PHF20 gene expression inhibitor or PHF20 protein activity inhibitor
10 g of isomerized sugar
5 g of mannitol
appropriate amount of purified water

According to a conventional method for preparing liquids, the above components are added to purified water to dissolve. Then, an appropriate amount of lemon flavor is added thereto. Then, the above components are mixed. Then, purified water is added thereto. The mixture is adjusted to 100 ml by adding purified water, and then filled in a brown bottle to sterilize to prepare liquids.

### Formulation Example 2. Preparation of food formulations

### 2-1. Preparation of health food

10 µg of PHF20 gene expression inhibitor or PHF20 protein activity inhibitor appropriate amount of vitamin mixture
70 g of vitamin A acetate
1.0 mg of vitamin E
0.13 mg of vitamin B 1
0.15 mg of vitamin B2
0.5 mg of vitamin B6
0.2 g of vitamin B12
10 mg of vitamin C
10 g of biotin
1.7 mg of nicotinamide
50 g of folic acid
0.5 mg of calcium pantothenate
appropriate amount of mineral mixture
1.75 mg of ferrous sulfate
0.82 mg of zinc oxide
25.3 mg of magnesium carbonate
15 mg of potassium monophosphate
55 mg of dibasic calcium phosphate
90 mg of potassium citrate
100 mg of calcium carbonate
24.8 mg of magnesium chloride

The mixing ratio of the above vitamin and mineral is relatively suitable for health food in a preferred embodiment, but the mixing ratio may be arbitrarily modified. After mixing the above ingredients according to a conventional health food manufacturing method, granules are prepared and may be used for a method of preparing the health food composition according to a conventional method.

### 2-2. Preparation of health drinks

10 µg of PHF20 gene expression inhibitor or PHF20 protein activity inhibitor
15 g of vitamin C
100 g of vitamin E (powder)
19.75 g of iron lactate
3.5 g of zinc oxide
3.5 g of nicotinamide
0.2 g of vitamin A
0.25 g of vitamin B 1
0.3 g of vitamin B2
certain amount of water

After mixing the above ingredients according to the conventional method of preparing health drink, they are stirred and heated at 85°C for about 1 hour. The resulting solution is filtered and placed in a sterilized 2 L container. It is sealed and sterilized, and then refrigerated. It is used to prepare the health drink composition of the invention.

Although the composition ratio is prepared by mixing ingredients suitable for relatively favorite beverages in a preferred embodiment, the mixing ratio may be arbitrarily modified according to regional and national preferences such as demanding class, demanding country, and use.

### Formulation Example 3. Preparation of feed additives

PHF20 gene expression inhibitor or PHF20 protein activity inhibitor 2.0%
glucose 2.0%
peptone 1.0%
yeast extract 1.0%
diphosphate 0.2%
magnesium sulfate 0.05 %
cysteine 0.05%
purified water to 100 %

As an excipient, defatted rice bran appropriate amount

Above, a specific part of the present invention has been described in detail, but it is apparent for those of ordinary skill in the art that this specific description is only a preferred embodiment, and the scope of the present invention is not limited thereby. Accordingly, it is intended that the substantial scope of the present invention be defined by the appended claims and their equivalents.

## Claims

1. A pharmaceutical composition for preventing or treating diseases caused by muscle loss, the composition comprising a PHD finger protein 20 (PHF20) gene expression inhibitor or PHF20 protein activity inhibitor as an active ingredient.

2. The pharmaceutical composition of claim 1, wherein the disease caused by the muscle loss is selected from the group consisting of sarcopenia, atony, muscular atrophy, muscular dystrophy, muscle degeneration, myotonic dystrophy, amyotrophic lateral sclerosis, myasthenia, and cachexia.

3. The pharmaceutical composition of claim 1, wherein the PHF20 gene expression inhibitor includes at least one type selected from the group consisting of shRNA, siRNA, ribozyme, and antisense nucleotides complementary to the PHF20 gene.

4. The pharmaceutical composition of claim 3, wherein the shRNA is represented by the nucleotide sequence of SEQ ID NO: 1.

5. The pharmaceutical composition of claim 1, wherein the PHF20 gene expression inhibitor is a Yin Yang 1 (YY1) promoter activity inhibitor.

6. The pharmaceutical composition of claim 1, wherein the PHF20 gene expression inhibitor increases the expression of MyoD (myogenic differentiation marker) to promote myogenic differentiation.

7. The pharmaceutical composition of claim 1, wherein the PHF20 protein activity inhibitor includes at least one type selected from the group consisting of a compound, peptide and antibody that specifically binds to PHF20 protein.

8. A pharmaceutical composition for preventing or treating diseases caused by muscle loss, the composition comprising PHF20 gene expression inhibitor or PHF20 protein activity inhibitor; or YY1 promoter activity inhibitor.

9. A health functional food composition for increasing muscle or inhibiting muscle loss, the composition comprising a PHF20 gene expression inhibitor or a PHF20 protein activity inhibitor as an active ingredient.

10. A feed additive composition for increasing muscle or inhibiting muscle loss, the composition comprising a PHF20 gene expression inhibitor or a PHF20 protein activity inhibitor as an active ingredient.

11. A method of treating a disease caused by muscle loss, the method comprising the step of treating an individual in need thereof with a PHF20 (PHD finger protein 20) gene expression inhibitor or a PHF20 protein activity inhibitor.


SEQUENCE LISTING

<110>  The Industry & Academic Cooperation in Chungnam National
       University (IAC)

<120>  Composition for prevention or treatment of disease caused by
       muscle loss comprising expression inhibitors of PHF20

<130>  CNU1.3PCT

<150>  KR10-2020-0070987
<151>  2020-06-11

<160>  13

<170>  PatentIn version 3.2

<210>  1
<211>  28
<212>  RNA
<213>  shPHF20

<400>  1
ccggcagugu cagagugugg uaaagcuc                                          28


<210>  2
<211>  19
<212>  RNA
<213>  siYY1

<400>  2
ccaaacaacu ggcagaauu                                                    19


<210>  3
<211>  21
<212>  RNA
<213>  control shRNA

<400>  3
uucuccgaac gugucacguu u                                                 21


<210>  4
<211>  18
<212>  DNA
<213>  mouse GAPDH forward

<400>  4
tccagtatga ctccactc                                                     18


<210>  5
<211>  18
<212>  DNA
<213>  mouse GAPDH reverse

<400>  5
atttctcgtg gttcacac                                                     18


<210>  6

<211>  21
<212>  DNA
<213>  mouse PHF20 forward

<400>  6
cattgactac gaagaaggga g                                                21


<210>  7
<211>  21
<212>  DNA
<213>  mouse PHF20 reverse

<400>  7
cttctctaaa gggcgcagat a                                                21


<210>  8
<211>  25
<212>  DNA
<213>  mouse YY1 forward

<400>  8
cagaagcagg tgcagatcag accct                                            25


<210>  9
<211>  21
<212>  DNA
<213>  mouse YY1 reverse

<400>  9
gcaccaccac ccacggaatc g                                                21


<210>  10
<211>  20
<212>  DNA
<213>  mouse MyoD forward

<400>  10
tacagtggcg actcagatgc                                                  20


<210>  11
<211>  20
<212>  DNA
<213>  mouse MyoD reverse

<400>  11
gagatgcgct ccactatgct                                                  20


<210>  12
<211>  18
<212>  DNA
<213>  mouse YY1(-200/-55) forward

<400>  12
cgctgccttc ctccctct                                                    18


<210>  13

<211>  18
<212>  DNA
<213>  mouse YY1(-200/-55) reverse

<400>  13
cgtccgtggc gatgtaga                                                    18